Europäisches Patentamt

(19) European Patent Office

Office européen des brevets

(11) Publication number : **0 010 412**
**B2**

# (12) NEW EUROPEAN PATENT SPECIFICATION

(45) Date of publication of the new patent specification :
11.11.87

(51) Int. Cl.⁴ : **A 61 K   7/30**

(21) Application number : 79302199.9

(22) Date of filing : 12.10.79

(54) Denture cleansing composition and process for its preparation.

(30) Priority : 13.10.78 ZA 785772

(43) Date of publication of application :
30.04.80 Bulletin 80/09

(45) Publication of the grant of the patent :
03.11.82 Bulletin 82/44

(45) Mention of the opposition decision :
11.11.87 Bulletin 87/46

(84) Designated contracting states :
AT BE CH DE FR GB IT LU NL SE

(56) References cited :
AT-A-   264 015
CH-A-   465 114
DE-A- 2 025 338
DE-A- 2 312 847
FR-A- 1 428 137
GB-A- 1 292 482
GB-A- 1 527 010
US-A- 3 607 759
The file contains technical information submitted
after the application was filed and not included in this
specification

(73) Proprietor : RECKITT AND COLMAN PRODUCTS LIMI-
TED
P.O. Box 26 1-17, Burlington Lane
London W4 2RW.(GB)

(72) Inventor : Bogie, Kenneth David
21, Skirbeck Road Gillshill Road James Reckitt Av.
Hull N. Humberside (GB)

(74) Representative : Oulton, Richard John et al
H.L. COTTRELL & CO. Kings Building South Church
Side
Hull North Humberside HU1 1RR. (GB)

EP 0 010 412 B2

## Description

This invention relates to denture cleansing compositions intended for dissolution in an aliquot of water to develop an initial pH of ≤ 4.5.

Denture cleansing compositions are known and generally comprise solids compositions which produce a more or less strongly alkaline reaction on dissolution in water to form a cleansing bath.

Tablets have been found to be an acceptable form of presentation, facilitating dosage control both in manufacture and use, improving ease of handling from a packaging point of view, and in storage. Tabletted alkaline denture cleansing compositions clean dentures but do not completely remove accumulations of calculus or tartar.

Known acid-reacting compositions, such as those containing hydrochloric acid, remove calculus or tartar but such compositions cannot be tabletted, require special handling and packaging, and are inconvenient in use so that their application to denture cleansing compositions generates many problems in both manufacture and use. Thus, no strongly acid-reacting denture cleansing tablet has yet been successfully marketed.

Austrian Patent Specification No. 264015 suggests the preparation of mixtures capable of dissolving in water to form a cleansing solution having an initial pH ≤ 5, preferably pH 1.5-4.5 and containing inter alia monopotassium permonosulphate, a carbonate of sodium and an acid reacting substance including sodium bisulphate or a fruit acid. Further it is proposed that certain of such mixtures be formed into tablets for dissolution in water to form a denture cleansing bath and suggested that the tablets may dissolve within 7 minutes.

The disclosure in Austrian Patent Specification No. 264015 does not however suggest how the mixtures are to be handled, and, more particularly, how the tablets are to be made on a commercial scale. AT-264015 does not suggest that moisture levels are of any importance and certainly does not mention or recognise that free surface moisture content is critical. Indeed the moisture content of the suggested components produces tablets which are not storage stable and the tablets take an inordinately long time to dissolve. Austrian Patent Specification No. 264015 makes no reference whatsoever to this use of named sodium carbonate in the acid reacting denture cleansing compositions described. Further, whilst tablets may be successfully produced by hand in accordance with the prior disclosure such processing is not commercially viable and does not produce a fast-dissolving tablet which can effectively remove calculus and generally clean dentures within ten minutes, a period to which the public has now become accustomed.

The invention as claimed is intended to provide an anhydrous denture cleansing tablet which, on dissolution in an aliquot of water, produces an acid-reacting bath having an initial pH of ≤ 4.5.

The present invention thus provides as claims.

The advantages offered by the invention are the provision of acid-reacting compositions which, contrary to the prior art acid-reacting compositions, are readily tabletted by conventional high speed tabletting apparatus. The tablets according to the invention dissolve in water in a very shor time (i. e. less than 5 minutes) and well within that expected by users and, are convenient to handle, package store and use in tablet form. In effect, the compositions provide the first acid-reacting compositions tabletted on a commercial scale.

Conveniently the composition may include up to 50 % of sodium bicarbonate, by weight based on the sodium carbonate.

Preferably the tartar removal component comprises up to 45 % by weight of the composition, conveniently from 2 % to 40 % by weight of the composition, and most preferably from 17 % to 28 % by weight of the composition.

Preferably at least part of the tartar removal component comprises tartaric acid and conveniently the amount of chloride-free anhydrous alkali metal carbonate present is a stoichiometrical amount to form with the tartaric acid, sodium hydrogen tartrate when the tablet is dissolved in water to form a denture cleansing bath.

The tablet may conveniently contain one or more additives selected from an antirestaining amount of disodium dihydrogen pyrophosphate, present at up to 10 % by weight of the tablet, the tablet may also conveniently contain anhydrous sodium perborate, present at from 2.5 % to 6.0 % by weight of the tablet, alone or admixed with anhydrous sodium carbonate and a minor amount of anionic surfactant such as sodium dodecylbenzene sulphonate, a disintegrant/binder material, present at from 2 % to 20 % by weight of the tablet, and at least one colourant, and/or flavourant, and/or tabletting aid each below 0.2 % by weight of the tablet.

The tablet in accordance with the invention, conveniently consists of a unit dose. The tablet of the invention is capable of dissolving in an aliquot of water preferably in less than 2 minutes.

The invention also envisages a process for the preparation of the anhydrous denture cleansing tablet in accordance with the invention characterised by the steps of predrying at least one component of the composition and dry mixing the dried component with the other components and tabletting to produce the denture cleansing tablet.

Thus, the process of this invention defines the steps for manufacturing the acid reacting denture cleansing tablets of the invention.

Preferably the said predried components are the tartar removal component and the anhydrous sodium carbonate.

When part of the anhydrous sodium carbonate is replaced by sodium bicarbonate, the bicarbonate is preferably added after the drying step.

In a preferred process in accordance with the invention the predried components are dried to a free surface moisture content of 0.02 % to 0.5 % by weight of the composition, preferably to a free surface moisture content $\leqslant 0.2$ % w/w.

Preferably the drying step is conducted on a fluid bed drier at 80 °C for 15 minutes.

In a preferred embodiment in accordance with the invention tablets are prepared by predrying sodium bisulphate and/or an organic acid to a moisture content $\leqslant 0.5$ % w/w and preferably 0.1 %-0.2 % w/w. A premix is made of the sodium bisulphate and/or tartaric acid and flavourant and colourant used. The balance of components is dry-mixed in any order with the pre-mix in a suitable mixer. The mixture is then tabletted in known manner.

The tablets according to the invention have been found to be storage-stable and even after long storage periods can produce a cleansing bath that removes calculus or tartar and stains together with the bulk of any bacterial infection yet has no harmful effects on the materials used in the manufacture of dental prostheses.

The invention is further illustrated by the following examples in which parts and percentages are by weight of the total composition except where otherwise stated :

## Example 1

Tartaric acid and a dense, granula form of anhydrous sodium carbonate of food grade containing less than 0.3 % of chloride were separately dried on a Calmic (Trade Mark) fluid bed drier for 15 minutes at 80 °C. Each product was found to have a free surface moisture content below 0.2 %.

A portion of tartaric acid was premixed in a small shear/diffusive mixer with 0.032 % of HEXACOL Indigo Carmine Supra dye, 0.082 % of peppermint flavourant and 0.033 % aniseed flavourant.

The pre-mix was transferred to a tumble mixer and the balance of tartaric acid and the other components admixed to give a final composition for tabletting :

| | |
|---|---|
| « Oxone » (Trade Mark) | 57.701 % |
| Tartaric acid (total) | 24.88 % |
| Anhydrous Sodium Carbonate | 12.27 % |
| Carbowax (Trade Mark) 6000 | 0.82 % |
| Anhydrous Sodium perborate « Ground Mix » | 4.182 % |
| Dyestuff (as above) | 0.032 % |
| Total flavourant (as above) | 0.115 % |

By weight of the « Ground Mix » the anhydrous sodium perborate « Ground Mix » consisted of 74.3 % anhydrous sodium perborate ground together with 25.5 % of anhydrous sodium carbonate and 0.2 % NANSA S80 (Trade Mark) to an intimately mixed fine powder.

The final composition for tabletting was fed to a MANESTY D3 press and was straight-forwardly formed into tablets of weight 3.0 g, diameter 22 mm and thickness 4 mm. Whilst the tablets were a little soft initially, they presented a good uniform smooth surface with a blue mottled appearance.

On placing one tablet in 100 ml tap water at 30 °C, dissolution was accompanied by vigorous effervescence and there was no trace of solids residue, within 50 seconds.

The pH of a clear 1 % solution was determined and found to be 3.4. Using more than one aliquot of water the pH was found to vary about 3.6 by plus or minus 0.3. This variation was attributed to varying aliquot sizes and is acceptable experimental error.

The cleansing bath produced was tested for efficacy using naturally soiled dentures bearing stains and accumulations of calculus or tartar. Almost complete elimination of such accumulations occurred with soaking for < 15 minutes and stains were simultaneously removed. Before reapplication, the dentures required a simple rinsing under running water from the tap. No ulceration was suffered by denture wearers in the test period.

A tendency to restain to a greyish shade was noted in some denture wearers. This was overcome by including a proportion of disodium dihydrogen pyrophosphate as shown in Example 4.

## Examples 2-6

The method of Example 1 was followed, where applicable substituting sodium bisulphate for the tartaric acid, using the following components :

(See Table page 4)

| | Ex 2 | 3 | 4 | 5 | 6 |
|---|---|---|---|---|---|
| "OXONE" (contains $KHSO_4 \cdot K_2SO_4 \cdot 2KHSO_5$) | 58.4 | 57.97 | 56.893 | 46.118 | 58.40 |
| Tartaric acid | — | 25.7 | 24.52 | — | 25.20 |
| Sodium bisulphate | 25.27 | — | — | 41.00 | — |
| Anhydrous sodium carbonate (chloride 0.3%) | 12.0 | 12.0 | 11.77 | 9.50 | 12.08 |
| Anhydrous sodium perborate "Ground Mix" | 4.196 | — | 4.05 | 3.28 | — |
| Disodium dihydrogen pyrophosphate | — | — | 2.64 | — | — |
| AVICEL PH100 (Trade Mark) | — | 4.196 | — | — | — |
| KELACID (Trade Mark) | — | — | — | — | 4.16 |
| HEXACOL INDIGO CARMINE SUPRA (Trade Mark) | 0.017 | 0.017 | 0.016 | 0.013 | 0.02 |
| Peppermint Flavour | 0.084 | 0.084 | 0.078 | 0.063 | 0.1 |
| Aniseed Flavour | 0.033 | 0.033 | 0.033 | 0.026 | 0.04 |

Wheras anhydrous sodium perborate « Ground Mix » is included, it is present as a disintegrant and has been replaced respectively by AVICEL PH100 (Trade Mark) and KELACID (Trade Mark) in Examples 3 and 6. The anhydrous sodium perborate « Ground Mix » may well as a tabletting aid auxiliary ; certain of the replacements are known tablet binder aids cum disintegrants.

All these compositions were found to be effective in dissolving in water at 20 °C to 50 °C to a clear solution, of pH 3.4 to 4.0 that remove calculus or tartar accumulations. Additionally, a bacterial count before and after use of tablets according to Examples 1 and 4 in cleansing baths for dentures showed marked reduction after cleansing.

If more than one tablet is used to produce a clear solution then correspondingly more water is required.

These tablets, all containing the same dyestuff produce an initially blue coloured cleansing bath which colour fades after about 2-8 minutes depending on the water bath temperature and the bleaching action of the monopotassium monopersulphate-containing triple salt.

The term « free surface moisture content » is used herein to define that part of the moisture in the composition available for reaction, e. g. with calcium carbide, to form acetylene, and includes some capillary water and loosely bound water. Such moisture content may conveniently be measured by the well known modified Karl-Fisher Test or the moisture evolution analyser of DuPont.

It has been found that unless the proper free surface moisture content of the composition as defined in the appended claims is realised, the tablets produced may be defective, exhibiting star cracks in the surfaces and a tendency to « cap », i. e. laminate, during pressing even.

By substantially « chloride-free » as used herein is meant that the carbonate contains no more than trace amounts of chloride as impurity. Larger concentrations of chloride would be released at least in part as chloride during storage, thus imparing the quality of the tablets and reducing their oxidising power. Additionally, excessive residual chlorine would be released in the denture cleansing bath producing malodour and objectionable taste when cleaned dentures are worn by some users.

The sodium carbonate is preferably a dense and granular form of anhydrous sodium carbonate containing less than 0.3 % w/w of chloride.

**Claims** (for the Contracting States : BE, CH, DE, FR, GB, IT, LU, NL, SE)

1. An anhydrous denture cleansing tablet for dissolution in an aliquot of water to form a denture cleansing bath of pH $\leq$ 4.5 including a permonosulphate, a tartar removal component and a carbonate characterised in that the tablet is capable of dissolving in less than 5 minutes ; the permonosulphate is a triple salt, $KHSO_4$. $K_2SO_4$. $2KHSO_5$ and is present in an amount sufficient to provide from 10 to 40 % by weight of the composition of $KHSO_5$ ; the tartar removal component comprises sodium bisulphate and/or tartaric acid ; the carbonate is substantially chloride-free, anhydrous sodium carbonate, $Na_2CO_3$, which is present in sufficient amount to develop an initial pH $\leq$ 4.5 in the bath ; and the free surface moisture content of the composition for tabletting is $\leq$ 0.2 % by weight of the composition.

2. A tablet as claimed in Claim 1 characterised in that up to 50 % by weight of the carbonate in the tablet is replaced by sodium bicarbonate.

3. A tablet as claimed in any preceding claim characterised in that the tartar removal component comprises up to 45 % by weight of the composition.

**0 010 412**

4. A tablet as claimed in any preceding claim characterised in that the tablet includes tartaric acid and sodium carbonate in stoichiometric amount to form sodium hydrogen tartrate when the tablet dissolves.

5. A tablet as claimed in Claim 1 characterised in that it contains one or more additives selected from an anti-restaining amount of disodium dihydrogen pyrophosphate present at up to 10 % by weight of the tablet, anhydrous perborate present at from 2.5 % to 6.0 % by weight of the tablet alone or mixed with anhydrous sodium carbonate and a minor amount of anionic surfactant such as sodium dodecylbenzene sulphonate, a disintegrant/binder material present at from 2 % to 20 % by weight of the tablet, and at least one colourant, and/or flavourant, and/or tabletting aid each below 0.2 % by weight of the tablet.

6. A process for the preparation of an anhydrous denture cleansing tablet as claimed in any one of Claims 1 to 5, characterised by the steps of predrying at least one component of the tablet, dry mixing the dried component with the other components and tabletting the composition.

7. A process as claimed in Claim 6 characterised in that the predried components are the tartar removal component and the anhydrous sodium carbonate.

8. A process as claimed in Claim 6 or 7, characterised in that the predried components are dried to a free moisture content of 0.02 % to 0.5 % by weight of the tablet.

9. A process as claimed in Claim 6, 7 or 8 characterised in that the predried components are dried to a free surface moisture content of $\leq 0.2$ % w/w.

10. A process as claimed in any one of Claims 6 to 9 inclusive characterised in that the drying step is conducted on a fluid bed drier at 80 °C for 15 minutes.


**Claims** (for the Contracting State AT)

1. A process for the preparation of an anhydrous denture cleansing tablet for dissolution in an aliquot of water to form a denture cleansing bath of pH $\leq 4.5$, said composition including a permonosulphate, a tartar removal component and a carbonate characterised in that the tablet is capable of dissolving in less than 5 minutes ; the permonosulphate is a triple salt $KHSO_4. K_2SO_4. 2KHSO_5$ and is triple salt being present in an amount sufficient to provide from 10 to 40 % by weight of the composition of $KHSO_5$ ; the tartar removal component comprises sodium bisulphate and/or tartaric acid ; the carbonate is substantially chloride-free, anhydrous sodium carbonate, $Na_2CO_3$, which is present in sufficient amount to develop an initial pH $\leq 4.5$ in the bath ; and characterised by the steps of pre-drying at least one component in the composition and dry mixing the dried components with the other components to produce a composition with a free surface moisture content $\leq 0.2$ % by weight of the composition, and subjecting the mixture to tabletting.

2. A process as claimed in claim 1 characterised by the step of pre-drying the selected component or components to obtain a free surface moisture content of 0.02 % to 0.5 % of the tablet.

3. A process as claimed in claim 2 characterised in that the pre-dried component or components are dried to a free surface moisture content of $\leq 0.2$ % of the tablet.

4. A process as claimed in any one of claims 1 to 3 characterised in that the tartar removal component comprises up to 45 % by weight of the tablet.

5. A process as claimed in any one of claims 1 to 4 characterised by the step of mixing sodium bicarbonate with the sodium carbonate in an amount of up to 50 % by weight of the sodium carbonate, after the drying step.

6. A process as claimed in any one of claims 1 to 5 characterised in that the drying step is conducted on a fluid bed drier at 80 °C for 15 minutes.


**Patentansprüche** (für die Vertragsstaaten : BE, CH, DE, FR, GB, IT, LU, NL, SE)

1. Wasserfreie Zahnreinigungstablette zum Auflösen in einer aliquoten Menge Wassers zur Bildung eines Zahnreinigungsbades vom pH $\leq 4,5$, enthaltend ein Permonosulfat, eine zahnsteinentfernende Komponente und ein Carbonat, dadurch gekennzeichnet, daß die Tablette in weniger als fünf Minuten auflösbar ist, daß das Permonosulfat ein Tripelsalz, $KHSO_4.K_2SO_4.2KHSO_5$, ist und in einer ausreichenden Menge vorhanden ist, um 10 bis 40 Gew.-% $KHSO_5$ der Zusammensetzung auszumachen, daß die zahnsteinentfernende Komponente Natriumbisulfat und/oder Weinsäure enthält, das Carbonat im wesentlichen chloridfreies, wasserfreies Natriumcarbonat $Na_2CO_3$ ist, welches in einer ausreichenden Menge vorhanden ist, um ein Anfangs-pH $\leq 4,5$ im Bad zu entwickeln, und daß der freie Oberflächenfeuchtigkeitsgehalt der zu tablettierenden Zusammensetzung $\leq 0,2$ Gew.-% der Zusammensetzung beträgt.

2. Tablette nach Anspruch 1, dadurch gekennzeichnet, daß bis zu 50 Gew.-% des Carbonats in der Tablette durch Natriumbicarbonat ersetzt ist.

3. Tablette nach einem der vorhergehenden Ansprüche, dadurch gekennzeichnet, daß die zahnsteinentfernende Komponente bis zu 45 Gew.-% der Zusammensetzung ausmacht.

4. Tablette nach einem der vorhergehenden Ansprüche, dadurch gekennzeichnet, daß die Tablette

5

Weinsäure und Natriumcarbonat in stöchiometrischer Menge enthält, um beim Auflösen der Tablette Natriumhydrogentartrat zu bilden.

5. Tablette nach Anspruch 1, dadurch gekennzeichnet, daß sie einen oder mehrere Zusätze enthält, ausgewählt aus einer Rückverfärbungen verhindernden Menge Dinatriumdihydrogenpyrophosphat von bis zu 10 Gew.-% der Tablette, wasserfreiem Perborat in 2,5 bis 6,0 Gew.-% der Tablette allein oder vermischt mit·wasserfreiem Natriumcarbonat und einer geringen Menge eines anionischen Netzmittels, wie Natriumdodecylbenzolsulfonat, einem Sprengmittel/Bindemittel in einer Menge von 2 bis 20 Gew.-% der Tablette, und mindestens einem Farbstoff und/oder Geschmacksstoff und/oder Tablettierungshilfsmittel jeweils unter 0,2 Gew.-% der Tablette.

6. Verfahren zur Herstellung einer wasserfreien Zahnreinigungstablette nach einem der Ansprüche 1 bis 5, gekennzeichnet durch die Schritte des Vortrocknens zumindest einer Komponente der Tablette, des trockenen Vermischens der getrockneten Komponente mit den anderen Komponenten und des Tablettierens der Zusammensetzung.

7. Verfahren nach Anspruch 6, dadurch gekennzeichnet, daß die vorgetrockneten Komponenten die zahnsteinentfernende Komponente und das wasserfreie Natriumcarbonat sind.

8. Verfahren nach Anspruch 6 oder 7, dadurch gekennzeichnet, daß die vorgetrockenten Komponenten bis zu einem freien Feuchtigkeitsgehalt von 0,02 Gew.-% bis 0,5 Gew.-% der Tablette getrocknet werden.

9. Verfahren nach Anspruch 6, 7 oder 8, dadurch gekennzeichnet, daß die vorgetrockneten Komponenten bis zu einem freien Oberflächenfeuchtigkeitsgehalt von 0,2 Gew.-% getrocknet werden.

10. Verfahren nach einem der Ansprüche 6 bis 9, dadurch gekennzeichnet, daß der Trocknungsschritt in einem Wirbelschichttrockner 15 Minuten lang bei 80 °C durchgeführt wird.

**Patentansprüche** (für den Vertragsstaat AT)

1. Verfahren zur Herstellung einer wasserfreien Zahnreinigungstablette zum Auflösen in einer aliquoten Menge Wassers zur Bildung eines Zahnreinigungsbades vom pH $\leq 4,5$, enthaltend ein Permonosulfat, eine zahnsteinentfernende Komponente und ein Carbonat, dadurch gekennzeichnet, daß die Tablette in weniger als fünf Minuten auflösbar ist, daß das Permonosulfat ein Tripelsalz, $KHSO_4 \cdot K_2SO_4 \cdot 2KHSO_5$, ist und in einer ausreichenden Menge vorhanden ist, um 10 bis 40 Gew.-% $KHSO_5$ der Zusammensetzung auszumachen, daß die zahnsteinentfernende Komponente Natriumbisulfat und/oder Weinsäure enthält, das Carbonat im wesentlichen chloridfreies, wasserfreies Natriumcarbonat $Na_2CO_3$ ist, welches in einer ausreichenden Menge vorhanden ist, um ein Anfangs-pH $\leq 4,5$ im Bad zu entwickeln, und gekennzeichnet durch die Schritte des Vortrocknens zumindest einer Komponente der Zusammensetzung und des trockenen Vermischens der getrockneten Komponenten mit den anderen Komponenten unter Bildung einer Zusammensetzung mit einem freien Oberflächenfeuchtigkeitsgehalt von $\leq 0,2$ Gew.-% der Zusammensetzung, und des Tablettierens des Gemisches.

2. Verfahren nach Anspruch 1, gekennzeichnet durch den Schritt des Vortrocknens der gewählten Komponente(n) zu einem freien Oberflächenfeuchtigkeitsgehalt von 0,02 Gew.-% bis 0,5 Gew.-% der Tablette.

3. Verfahren nach Anspruch 2, dadurch gekennzeichnet, daß die vorgetrocknete(n) Komponente(n) bis zu einem freien Oberflächenfeuchtigkeitsgehalt von $\leq 0,2$ Gew.-% der Tablette getrocknet werden.

4. Verfahren nach einem der Ansprüche 1 bis 3, dadurch gekennzeichnet, daß die zahnsteinentfernende Komponente bis zu 45 Gew.-% der Tablette umfaßt.

5. Verfahren nach einem der Ansprüche 1 bis 4, gekennzeichnet durch den Schritt des Vermischens von Natriumbicarbonat mit dem Natriumcarbonat in einer Menge von bis zu 50 Gew.-% des Natriumcarbonats nach dem Trocknungsschritt.

6. Verfahren nach einem der Ansprüche 1 bis 5, dadurch gekennzeichnet, daß der Trocknungsschritt in einem Wirbelschichttrockner 15 Minuten lang bei 80 °C durchgeführt wird.

**Revendications** (pour les Etats contractants : BE, CH, DE, FR, GB, IT, LU, NL, SE)

1. Comprimé anhydre de nettoyage de dentiers destiné à être dissous dans une partie aliquote d'eau pour former un bain de nettoyage de dentiers d'un pH $\leq 4,5$, comprenant un permonosulfate, un composant éliminant le tartre et un carbonate, caractérisé en ce que le comprimé est susceptible de se dissoudre en moins de cinq minutes, en ce que le permonosulfate est un sel triple ($KHSO_4$, $K_2SO_5$, $2KHSO_5$) et est présent en une quantité suffisante pour constituer de 10 à 40 % en poids de la composition de $KHSO_5$, en ce que le composant éliminant le tartre est constitué par du bisulfate de sodium et/ou un acide tartrique, en ce que le carbonate est un carbonate de sodium anhydre pratiquement exempt de chlorure, $Na_2CO_3$, qui est présent en une quantité suffisante pour établir dans le bain un pH initial $\leq 4,5$, en ce que la teneur en humidité superficielle libre de la composition pour former des comprimés est $\leq 0,2$ % en poids de la composition.

2. Comprimé selon la revendication 1, caractérisé en ce qu'on remplace jusqu'à 50 % en poids du carbonate dans le comprimé par du bicarbonate de sodium.

3. Comprimé selon l'une quelconque des revendications précédentes, caractérisé en ce que le composant éliminant le tartre représente jusqu'à 45 % en poids de la composition.

4. Comprimé selon l'une quelconque des revendications précédentes, caractérisé en ce qu'il contient de l'acide tartrique et du carbonate de sodium en une quantité stœchiométrique pour former de l'hydrogénotartrate de sodium lorsque le comprimé se dissout.

5. Comprimé selon la revendication 1, caractérisé en ce qu'il contient un ou plusieurs additifs choisis parmi le dihydrogéno-pyrophosphate disodique en une quantité inhibant la réapparition des colorations, soit jusqu'à 10 % en poids du comprimé, le perborate de sodium anhydre en une quantité de 2,5 à 6 % en poids du comprimé, seul ou en mélange avec le carbonate de sodium anhydre et une faible quantité d'un agent tensio-actif anionique tel que le dodécylbenzènesulfonate de sodium, une matière constituée d'un agent désintégrant et d'un agent liant en une quantité de 2 à 20 % en poids du comprimé, ainsi qu'au moins un colorant et/ou un agent aromatisant et/ou un adjuvant de formation de comprimés, chacun en une quantité inférieure à 0,2 % en poids.

6. Procédé de préparation du comprimé de nettoyage de dentiers selon l'une quelconque des revendications 1 à 5, caractérisé en ce qu'il comprend les étapes qui consistent à sécher préalablement au moins un composant du comprimé, à mélanger à sec le composant séché avec les autres composants et à transformer la composition en comprimés.

7. Procédé selon la revendication 6, caractérisé en ce que les composants préalablement séchés sont le composant éliminant le tartre et le carbonate de sodium anhydre.

8. Procédé suivant la revendication 6 ou 7, caractérisé en ce que les composants préalablement séchés sont séchés à une teneur en humidité libre de 0,02 % à 0,5 % en poids du comprimé.

9. Procédé suivant la revendication 6, 7 ou 8, caractérisé en ce que les composants préalablement séchés sont séchés à une teneur en humidité superficielle libre ≤ 0,2 % en poids/poids.

10. Procédé suivant l'une quelconque des revendications 6 à 9, caractérisé en ce que l'étape de séchage est effectuée dans un sécheur à lit fluide à une température de 80 °C pendant 15 minutes.

**Revendications** (pour l'Etat contractant AT)

1. Procédé pour la préparation d'un comprimé anhydre de nettoyage de dentiers destiné à être dissous dans une partie aliquote d'eau pour former un bain de nettoyage de dentiers d'un pH ≤ 4,5, ce comprimé contenant un permonosulfate, un composant éliminant le tartre et un carbonate, caractérisé en ce que le comprimé est susceptible de se dissoudre en moins de cinq minutes, en ce que le permonosulfate est un sel triple ($KHSO_4$, $K_2SO_4$, $2KHSO_5$) et est présent en une quantité suffisante pour constituer de 10 à 40 % en poids de la composition de $KHSO_5$, en ce que le composant éliminant le tartre est constitué par du bisulfate de sodium et/ou un acide tartrique, en ce que le carbonate est un carbonate de sodium anhydre pratiquement exempt de chlorure, $Na_2CO_3$, qui est présent en une quantité suffisante pour établir dans le bain un pH initial ≤ 4,5, et caractérisé en ce qu'il comprend les étapes qui consistent à sécher préalablement au moins un composant de la composition, à mélanger à sec les composants séchés avec les autres composants pour former une composition avec une teneur en humidité superficielle libre ≤ 0,2 % en poids de la composition, et à transformer le mélange en comprimés.

2. Procédé selon la revendication 1, caractérisé par l'étape dans laquelle on sèche préalablement le(s) composant(s) sélectionné(s) pour obtenir une teneur en humidité superficielle libre de 0,02 % à 0,5 % du comprimé.

3. Procédé selon la revendication 2, caractérisé en ce que le(s) composant(s) préalablement séché(s) est(sont) séché(s) à une teneur en humidité superficielle libre ≤ 0,2 % du comprimé.

4. Procédé selon l'une quelconque des revendications 1 à 3, caractérisé en ce que le composant éliminant le tartre représente jusqu'à 45 % en poids du comprimé.

5. Procédé selon l'une quelconque des revendications 1 à 4, caractérisé par l'étape dans laquelle on mélange le bicarbonate de sodium avec le carbonate de sodium en une quantité pouvant atteindre 50 % en poids du carbonate de sodium, après l'étape de séchage.

6. Procédé selon l'une quelconque des revendications 1 à 5, caractérisé en ce que l'étape de séchage est effectuée dans un sécheur à lit fluide à une température de 80 °C pendant 15 minutes.